# EUROPEAN PATENT SPECIFICATION

(11) **EP 1 330 254 B1**
(45) Date of publication and mention of the grant of the patent: **06.07.2005**
(21) Application number: 01974510.8
(22) Date of filing: 12.10.2001
(51) Int. Cl.: A61K 35/56, A61K 38/17, A61P 35/00

(54) **TREATMENT OF CANCERS BY APLIDINE IN CONJUNCTION WITH CARNITINE OR ACETYLCARNITINE**
BEHANDLUNG VON KREBSERKRANKUNGEN mit APLIDIN IN KOMBINATION MIT CARNITIN ODER ACETYLCARNITIN
TRAITEMENT DE CANCERS PAR APLIDINE EN ASSOCIATION AVEC DE LA CARNITINE OU ACETYL-CARNITINE

(30) Priority: 12.10.2000 GB 0025044; 13.10.2000 GB 0025209; 15.11.2000 WO PCT/GB00/04349; 23.03.2001 GB 0107373
(43) Date of publication of application: 30.07.2003
(73) Proprietor: PHARMA MAR, S.A., 28760 Tres Cantos, Madrid (ES)
(72) Inventor: LOPEZ LAZARO, Luis Pharma Mar, S.A., E-28760 Madrid (ES); FERNANDEZ SOUSA, José Maria Pharma Mar, S.A., E-28760 Madrid (ES); ARMAND, Jean-Pierre Institut Gustave-Roussy, F-94805 Villejuif Cedex (FR); RAYMOND, Eric Institut Gustave-Roussy, F-94805 Villejuif Cedex (FR)
(74) Representative: Ruffles, Graham Keith
(86) International application number: PCT/GB2001/004555
(87) International publication number: WO 2002/030441

(56) References cited:
- EP-A- 0 048 149
- WO-A-91/04985
- DATABASE BIOSIS [Online] BIOSCIENCES INFORMATION SERVICE, PHILADELPHIA, PA, US; ADY-VAGO, N. (1) ET AL: "L-carnitine as a protector against aplidine induced skeletal muscle toxicity." retrieved from STN XP001076672 & PROCEEDINGS OF THE AMERICAN ASSOCIATION FOR CANCER RESEARCH ANNUAL MEETING, (MARCH, 2001) VOL. 42, PP. 545. PRINT. MEETING INFO.: 92ND ANNUAL MEETING OF THE AMERICAN ASSOCIATION FOR CANCER RESEARCH NEW ORLEANS, LA, USA MARCH 24-28, 2001 ,
- FAIRCLOTH, G. (1) ET AL: "Schedule-dependency of aplidine, a marine depsipeptide with antitumor activity." PROCEEDINGS OF THE AMERICAN ASSOCIATION FOR CANCER RESEARCH ANNUAL MEETING, (MARCH, 1999) VOL. 40, PP. 394-395. MEETING INFO.: 90TH ANNUAL MEETING OF THE AMERICAN ASSOCIATION FOR CANCER RESEARCH PHILADELPHIA, PENNSYLVANIA, USA APRIL 10-14, 1999 AMERI, XP001002421
- FAIRCLOTH, G. (1) ET AL: "Aplidine (APL) is a novel marine-derived depsipeptide with in vivo antitumor activity." PROCEEDINGS OF THE AMERICAN ASSOCIATION FOR CANCER RESEARCH ANNUAL MEETING, (MARCH, 1998) VOL. 39, PP. 227. MEETING INFO.: 89TH ANNUAL MEETING OF THE AMERICAN ASSOCIATION FOR CANCER RESEARCH NEW ORLEANS, LOUISIANA, USA MARCH 28-APRIL 1, 1998 AMERICAN, XP001002422
- CHAPA A M ET AL: "Influence of intravenous L- carnitine administration in sheep preceding an oral urea drench." JOURNAL OF ANIMAL SCIENCE, (1998 NOV) 76 (11) 2930-7. , XP008002985

## Description

The present invention relates to the treatment of cancers using aplidine or related compounds which are aplidine analogs.

### BACKGROUND OF INVENTION

Cancer comprises a group of malignant neoplasms that can be divided into two categories, carcinoma, comprising a majority of the cases observed in the clinics, and other less frequent cancers, which include leukaemia, lymphoma, central nervous system tumours and sarcoma. Carcinomas have their origin in epithelial tissues while sarcomas develop from connective tissues and those structures that had their origin in mesoderm tissues. Sarcomas can affect, for instance, muscle or bone and occur in the bones, bladder, kidneys, liver, lung, parotid or spleen.

Cancer is invasive and tends to metastasise to new sites. It spreads directly into surrounding tissues and also may be disseminated through the lymphatic and circulatory systems. Many treatments are available for cancer, including surgery and radiation for localised disease, and drugs. However, the efficacy of available treatments on many cancer types is limited, and new, improved forms of treatment showing clinical benefit are needed. This is especially true for those patients presenting with advanced and/or metastatic disease. It is also true for patients relapsing with progressive disease after having been previously treated with established therapies for which further treatment with the same therapy is mostly ineffective due to acquisition of resistance or to limitations in administration of the therapies due to associated toxicities.

Chemotherapy plays a significant part in cancer treatment, as it is required for treatment of advanced cancers with distant metastasis and often helpful for tumour reduction before surgery, and many anti-cancer drugs have been developed based on various modes of action.

Dehydrodidemnin B, now known as aplidine, is the subject of WO91/04985. It is related to compounds known as didemnins, and has the following structure:

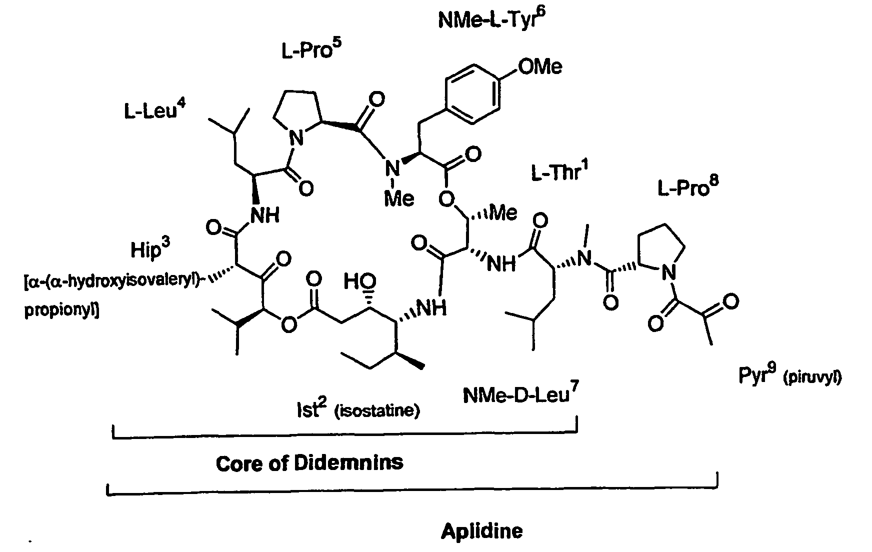

Further information on aplidine is to be found in, for example:
Jimeno, J., "Exploitation of marine microorganisms and invertebrates: Anticancer drugs from marine origin", IBC Conf Discov Drugs from Nat Novel Approaches New Sources (Dec 8-9, London) 1994.
Faircloth, G. et al., "Dehydrodidemnin B (DDM) a new marine derived anticancer agent (MDA) with activity against experimental tumour models", 9th NCI-EORTC Symp New Drugs Cancer Ther (March 12-15, Amsterdam) 1996, Abst 111
Sakai, R. et al., "Structure-activity relationships of the didemnins", Journal of Medicinal Chemistry 1996,39 (14): 2819
Urdiales, J.L. et al., "Antiproliferative effect of dehydrodidemnin B (DDB), a depsipeptide isolated from Mediterranean tunicates", Cancer Letters 1996, 102(1-2): 31
Faircloth, G. et al., "Preclinical characterization of aplidine (APD), a new marine anticancer depsipeptide (MADEP)", Proc Amer Assoc Cancer Res 1997, 38: Abst 692
Depenbrock, H. et al., "In vitro activity of aplidine, a new marine-derived anti-cancer compound, on freshly explanted clonogenic human tumour cells and haematopoietic precursor cells", British Journal of Cancer 1998, 78(6): 739
Faircloth, G. et al., "Aplidine (aplidine) is a novel marine-derived depsipeptide with in vivo antitumour activity", Proc Amer Assoc Cancer Res 1998, 39: Abst 1551
Faircloth, G. et al., "Preclinical development of aplidine, a novel marine-derived agent with potent antitumour activity", 10th NCI-EORTC Symp New Drugs Cancer Ther (June 16-19, Amsterdam) 1998, Abst 129
Mastbergen, S.C. et al., "Cytotoxicity and neurocytoxicity of aplidine, a new marine anticancer agent evaluated using in vitro assays", 10th NCI-EORTC Symp New Drugs Cancer Ther (June 16-19, Amsterdam) 1998, Abst 131

In preclinical studies, aplidine had dose-dependent cytotoxic activity against the two epithelial-like cell lines, CT-1 and CT-2, and the human colon cancer cell line, HT-29. The most proliferative line, CT-2, was the most sensitive to aplidine. In addition the compound decreased ornithine decarboxylase activity in all three cell lines (Lobo C, Garcia-Pozo SG, *et al*. Effect of dehydrodidemnin B on human colon carcinoma cell lines. Anticancer Research. 17: 333-336, Jan-Feb 1997). In a similar study, aplidine 50 nmol/L inhibited the growth of the breast cancer cell lines, MDA-MB231 and MCF-7 by 17 and 47%, respectively. A significant increase in spermidine and spermine was observed in the treated cells (Gomezfabre PM, Depedro E, et al. Polyamine contents of human breast cancer cells treated with the cytotoxic agents chlorpheniramine and dehydrodidemnin B. Cancer Letters. 113: 141-144, 26 Feb 1997). Flow cytometric analysis showed that aplidine did not induce any apparent cell cycle periubations (Erba E, Balconi G, et al. Cell cycle phases pertubations induced by new natural marine compounds. Annals of Oncology. 7 (Suppl. 1): 82, 1996). In mice, aplidine was active against implanted P388 leukaemia and B16 melanoma, with an optimal dose of 160 micrograms/kg. Unlike didemnin B, aplidine was active in SC implanted lewis lung carcinomas (Faircloth G, Rinehart K, *et al*. Dehydrodidemnin B a new marine derived anticancer agent with activity against experimental tumour models. Annals of Oncology. 7 (Suppl. 1): 34, 1996).

Continuous exposure to low concentrations of aplidine inhibited the growth of a number of tumour cell lines, including non-Hodgkin's lymphoma, melanoma and breast, melanoma, ovarian and non-small cell lung cancers. The magnitude of effect was dependent on the time of exposure and appeared to be achievable at non-myelotoxic concentrations. Non-small cell lung cancer, breast cancer and melanoma cell lines were sensitive to a continuous exposure to aplidine at concentrations of >= 0.001 micromol/L. Aplidine had similar toxicity to doxorubicin against clonogenic haematopoietic stem cells (Depenbrock H, Peter R, *et al*. In vitro activity of aplidine, a new marine-derived anti-cancer compound, on freshly explanted clonogenic human tumour cells and haematopoietic precursor cells. British Journal of Cancer. 78: 739-744, No. 6, Sep 1998).

Aplidine had significant activity against mice bearing human cancer xenografts. At a maximum tolerated dose of 2.1 mg/kg, aplidine produced near complete remissions in some animals with a treated/control (T/C) tumour ratio of 9%. At 1.25 mg/kg, significant activity was seen against gastric tumours (T/C 14%) and prostate tumour growth inhibition was also observed (T/C 25%) (Faircloth G, Grant W, *et al*. Preclinical development of aplidine, a novel marine-derived agent with potent antitumour activity. Annals of Oncology. 9 (Suppl. 2): 34, 1998).

Aplidine is related to other compounds of potential use against cancer, notably the didemnins. Aplidine is itself a dehydrodidemnin.

Examples of the related didemnins and other such compounds, which we generally refer to as aplidine analogues, are to be found in:
a) Rinehart KL, Kishore V, Bible KC, Sakai R, Sullins DW, Li KM. Didemnins and tunichlorin: novel natural products from the marine tunicate Trididemnum solidum.
   J Nat Prod. 1988 Jan-Feb;51(1):1-21.
   Erratum in:
   J Nat Prod 1988 May-Jun;51(3):624
b) Rinehart KL Jr, Gloer JB, Wilson GR, Hughes RG Jr, Li LH, Renis HE, McGovren JP.
   Antiviral and antitumor compounds from tunicates.
   Fed Proc. 1983 Jan;42(1):87-90.
c) Rinehart KL Jr, Gloer JB, Hughes RG Jr, Renis HE, McGovren JP, Swynenberg EB, Stringfellow DA, Kuentzel SL, Li LH.
   Didemnins: antiviral and antitumor depsipeptides from a caribbean tunicate.
   Science. 1981 May 22;212(4497):933-5.
d) Vervoort H, Fenical W, Epifanio RA.
   Tamandarins A and B: new cytotoxic depsipeptides from a
   Brazilian ascidian of the family Didemnidae.
   J Org Chem. 2000 Feb 11;65(3):782-92.
e) PCT/GB01/02901.
   Synthetic methods for aplidine and new antitumoral derivatives Filing Date 02 July 2001

The article (d) relates to aplidine analogues called tamandarines, notably tamandarine A and tamandarine B:

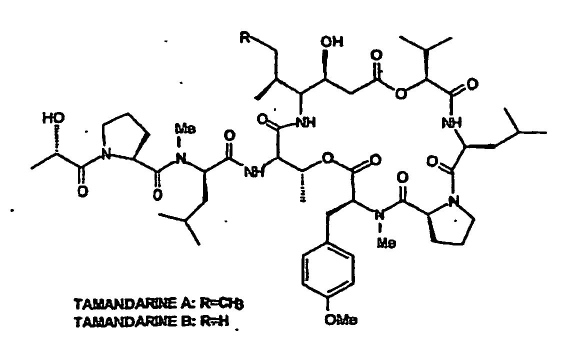

### Summary of Invention

We have developed improved medicaments to treat human patients with aplidine compounds, using muscle protectors such as L-carnitine. The aplidine compounds comprise aplidine itself, and aplidine analogues.

### EMBODIMENTS OF THE INVENTION

The present invention permits a method of treating any mammal, notably a human, affected by cancer which comprises administering to the affected individual a therapeutically effective amount of an aplidine compound, being aplidine or an aplidine analogue, or a pharmaceutical composition thereof, and a skeletal muscle protector, selected from L-carnitine, racemic carnitine, precursors and derivatives of L-carnitine, and acetylcarnitine.

The aplidine compound and muscle protector are usually administered as separate compositions with different dosing regimes.

The invention further allows a method of reducing the side effects of an aplidine compound, which involves administering a muscle protector such as L-carnitine. A method is also envisaged for enhancing the Recommended Dose of an aplidine compound, which involves administering a muscle protector.

The present invention relates to combination pharmaceutical preparations, which separately or together contain an aplidine compound and a skeletal muscle protector, selected from L-carnitine, racemic carnitine, precursors and derivatives of L-carnitine, and acetylcarnitine, as well as processes for their preparation. The combination preparations are for simultaneous or sequential use.

More particularly, the invention provides: the use of an aplidine compound in the preparation of a medicament for the treatment of a cancer by administering aplidine or an aplidine analogue and a skeletal muscle protector, selected from L-carnitine, racemic carnitine, precursors and derivatives of L-carnitine, and acetylcarnitine; the use of a skeletal muscle protector in the preparation of a medicament for the treatment of a cancer by administering aplidine or an aplidine analogue and a skeletal muscle protector, selected from L-carnitine, racemic carnitine, precursors and derivatives of L-carnitine, and acetylcamitine; and the use of an aplidine compound and a skeletal muscle protector in the preparation of a medicament for the treatment of a cancer by administering aplidine or an aplidine analogue and a skeletal muscle protector, selected from L-carnitine, racemic carnitine, precursors and derivatives of L-carnitine, and acetylcarnitine.

Examples of pharmaceutical compositions or medicaments include liquids (solutions, suspensions or emulsions) with suitable composition for intravenous administration, and they may contain the pure compound or in combination with any carrier or other pharmacologically active compounds.

Administration of an aplidine compound or the aplidine compositions of the present invention is based on a Dosing Protocol preferably by intravenous infusion. We prefer that infusion times of up to 72 hours are used, more preferably 1 to 24 hours, with about 1, about 3 or about 24 hours most preferred. Short infusion times which allow treatment to be carried out without an overnight stay in hospital are especially desirable. However, infusion may be around 24 hours or even longer if required. Infusion may be carried out at suitable intervals with varying patterns, illustratively once a week, twice a week, or more frequently per week, repeated each week optionally with gaps of typically one week.

Examples of dosing regimes with and without carnitine are given in the following table:

| Schedule | maximum tolerated dose, MTD | dose-limiting toxicity | recommended dose, RD |
|---|---|---|---|
| Aplidine weekly 24 hour infusion for 3 weeks, 1 week rest | 4500 | Muscular/ hepatic | 3750 |
| Aplidine weekly 1 hour infusion for 3 weeks, 1 week rest | 3600 | Muscular | 3250 |
| Aplidine 24 hour infusion every 2 weeks | 6000 | Muscular | 5000 |
| Aplidine 24 hour infusion every 2 weeks, L-carnitine daily | 8000 | Flu-like syndrome | 7000 |
| Aplidine 1-hour infusion for 5 consecutive days every 3 weeks | 1500 x 5 | Muscular/long lasting emesis | 1350 x 5 |

The correct dosage of the compound will vary according to the particular formulation, the mode of application, and the particular *situs*, host and tumour being treated. Other factors like age, body weight, sex, diet, time of administration, rate of excretion, condition of the host, drug combinations, reaction sensitivities and severity of the disease shall be taken into account. Administration can be carried out continuously or periodically within the maximum tolerated dose.

The preferred aplidine compound is aplidine, though the invention can be employed for the administration of aplidine analogues including those described in the documents (a) to (e) mentioned in the introduction. These documents are specificially incorporated herein by reference. Examples of the aplidine analogues include didemnin A, didemnin B, didemnin C, didemnin D and didemnin E, as well as all the compounds prepared in the PCT/ GB01 / 02901.

The preferred muscle protector is L-carnitine, though racemic carnitine, precursors and derivatives of L-carnitine can be employed. Examples of precursors and derivatives include acetylcarnitine and other esters of carnitine and fatty acids or other organic acids. Suitable dosages include 0.05 to 0.2 g/kg, more suitably 0.075 to 0.15 g/kg, preferably about 0.1 g/kg L-carnitine/day. These dosages can be varied as appropriate to suit other muscle protectors. It is convenient to administer the L- carnitine or other muscle protector in 3 divided portions, though other dosing regimes can be employed. In one currently preferred procedure, the dose of L-carnitine is not less than 3.5 g/day, such as 1.5 g three times a day.

As well as adminstering a muscle protector, the aplidine compound and compositions of this invention may be used with other drugs to provide a combination therapy. The other drugs may form part of the same composition as the aplidine, or be provided as a separate composition for administration at the same time or a different time. The identity of the other drug is not particularly limited, and suitable candidates include:
a) drugs with antimitotic effects, especially those which target cytoskeletal elements, including microtubule modulators such as taxane drugs (such as taxol, paclitaxel, taxotere, docetaxel), podophylotoxins or vinca alkaloids (vincristine, vinblastine);
b) antimetabolite drugs (such as 5-fluorouracil, cytarabine, gemcitabine, purine analogues such as pentostatin, methotrexate);
c) alkylating agents or nitrogen mustards (such as nitrosoureas, cyclophosphamide or ifosphamide);
d) drugs which target DNA such as the antracycline drugs adriamycin, doxorubicin, pharmorubicin or epirubicin;
e) drugs with target topoisomerases such as etoposide;
f) hormones and hormone agonists or antagonists such as estrogens, antiestrogens (tamoxifen and related compounds) and androgens, flutamide, leuprorelin, goserelin, cyprotrone or octreotide;
g) drugs which target signal transduction in tumour cells including antibody derivatives such as herceptin;
h) alkylating drugs such as platinum drugs (cis-platin, carbonplatin, oxaliplatin, paraplidineatin) or nitrosoureas;
i) drugs potentially affecting metastasis of tumours such as matrix metalloproteinase inhibitors;
j) gene therapy and antisense agents;
k) antibody therapeutics;
l) other bioactive compounds of marine origin, notably kahalalide F or the ecteinascidins such as et-743;
m) other drugs which combat side effects of aplidine such as antiemetics;
o) more generally drugs which allow aplidine to be dosed at the Recommended Dose and manage toxicity.

We have further found that aplidine inhibits expression of the gene (FLT1) encoding the receptor of the Vascular Endothelial Growth Factor (VEGF). In addition, aplidine has been found to severely inhibit production of the VEGF protein itself by tumour cells.

VEGF secretion by a cell mass, in particular a tumour cell mass, causes *de novo* vascularization (angiogenesis) leading to new blood vessels forming towards the cell mass and establishing a network of capillaries that is able to supply it with irrigation for its sustained proliferation. These effects, in particular the demonstrated abolition of production of VEGF by tumour cells are expected to severely inhibit the ability of the tumour cells to bring forth angiogenesis. In addition, VEGF is required directly by some hematopoietic tumour cells (such as MOLT4 human leukaemia cells) as a growth factor.

Thus aplidine can be predicted to have an inhibitory effect on *de novo* vascularization of growing primary tumours or metastases, therefore inhibiting growth of the tumours, which are known to require vascularization for growth. Aplidine should also be active on hematopoietic tumours.

Bladder tumours are one type of tumour over-expressing the receptor to Epithelial Growth Factor (EGF), which leads to upregulation of VEGF and the VEGF receptor. Binding of VEGF to its receptor is believed to lead to cell growth stimulation by means of transitory local calcium ion changes among other mechanisms for signalling. A compound inhibiting VEGF action is expected to be inhibitory to such tumours.

Experimentally, aplidine has been found to have exceedingly high activity on human bladder cancer (giving complete remissions in some animal models), in accordance with the prediction.

Aplidine can be predicted to have a broad spectrum antitumour activity due to its effects on a large number of tumours.

The effect of VEGF is more relevant because it involves an inhibition of new blood vessels. In addition to effects on blood vessels, certain tumours required VEGF directly for cell growth (i.e. leukaemia, lymphomas, bladder tumours and ovarian tumours).

Responses in cancer patients have been observed in clinical trials with aplidine, demonstrating usefulness of the method of treatment.

Phase I clinical studies and pharmacokinetic analysis demonstrate that aplidine presents a positive therapeutic window with manageable toxicity in the range of dosage required for clinical efficacy in the treatment of cancer patients. In particular, the present invention is expected to be of benefit for treatment of renal cancer, melanoma, medullary thyroid carcinoma, lung neuroendocrine tumors, non-Hodgkin lymphoma, colorectal cancer, non-small cell lung cancer, among others.

The method consists of administration of drug by intravenous infusion over a period of 72 hrs or less at the recommended dose level (RD) with or without combination with other therapeutic agents, in conjunction with the administration of a muscle protector.

Aplidine is supplied and stored as a sterile lyophilised product, consisting of aplidine and excipient in a formulation adequate for therapeutic use.

Solubilised aplidine shows substantial degradation under heat and light stress testing conditions, and a lyophilised dosage form was developed, see WO99/42125 incorporated herein by reference. In a currently preferred embodiment freeze-drying was performed from a 500 mg/mL solution of aplidine in 40% (v/v) *tert*-butanol in Water for Injection (WfI) containing 25 mg/mL D-mannitol as bulking agent. The prototype, containing 500 mg aplidine and 25 mg D-mannitol as bulking agent per vial was found to be the optimal formulation in terms of solubility, length of lyophilisation cycle and dosage requirements in the clinical studies. The optimal reconstitution solution was found to be 15/15/70% (v/v/v) Cremaphor EL/ethanol/WfI (CEW). Both reconstituted product and dilutions (up to 1:100 v/v) of the reconstituted product with normal saline appeared to be stable for at least 24 hours after preparation. Shelf-life data, available thus far, show that the formulation is stable for at least 1 year when stored at 4°C in the dark.

Preparation of the infusion solution is also performed under aseptic conditions by withdrawing the reconstituted solution volume corresponding to dosage calculated for each patient, and slowly injecting the required reconstituted solution volume into an infusion bag or bottle containing between 100 and 1000 ml of 0.9% sodium chloride, after which the whole is homogenised by slow manual shaking.

The aplidine infusion solution should be administered intravenously, as soon as possible, within 48 hours after preparation. PVC and polyethylene infusion systems, as well as clear glass are preferred container and conduit materials.

The administration is performed in cycles, in the preferred application method, an intravenous infusion of aplidine is given to the patients the first week of each cycle, the patients are allowed to recover for the remainder of the cycle. The preferred duration of each cycle is of either 3 or 4 weeks; multiple cycles can be given as needed. The drug may also be administered each of the first days of each cycle. Dose delays and/or dose reductions and schedule adjustments are performed as needed depending on individual patient tolerance of treatments, in particular dose reductions are recommended for patients with higher than normal serum levels of liver transaminases or alkaline phosphatase, or bilrubin.

The Recommended Dose (RD) is the highest dose which can be safely administered to a patient producing tolerable, manageable and reversibly toxicity according to the Common Toxicity Criteria established by the National Cancer Institute, (USA) with no more than 2 out of 6 patients presenting any dose limiting toxicities (DLT). Guidelines for cancer therapy frequently call for administration of chemotherapeutic agents at the highest safe dose at which toxicity is manageable in order to achieve maximum efficacy (DeVita, V.T. Jr., Hellman, S. and Rosenberg, S.A., Cancer: Principles and Practice of Oncology, 3rd ed., 1989, Lipincott, Philadelphia).

DLTs for aplidine using this method of treatment were determined in clinical studies. These studies established a recommended dose level for differing kinds of dosing protocols.

Aplidine can be safely administered at a dosage level at or below the Recommended Dose (RD).

Infusion is currently the preferred procedure, with typical regimes including the following:
24 hour infusion weekly for a number of weeks, say three weeks, followed by one week rest;
biweekly 24 hour infusion;
1 hour infusion weekly for 3 weeks every 4 weeks;
daily infusion of say 1 hour x 5 days q 3 weeks; and
infusion of say 3 hours every other week.

In particular, reference is made to the Examples and related discussion in our copending WO 0135974.

Previously the principal biological responses reported to the administration of aplidine had been observed in animal or in vitro models, known to be notoriously inaccurate concerning their usefulness to predict responses in human patients, or in human patients in experimental settings where an effective, safe method of treatment was unavailable (either the dosage used was a toxic dose significantly elevated over the recommended dose or the administration schedule was not appropriate).

In clinical trials using the method of this invention, appropriate plasma levels were achieved in patients at RD, and most importantly, objectively measurable responses demonstrated evidence of clinical benefit to patients.

Definitions for patient toxicities are adopted from WHO Criteria and the responses determined following WHO Response Criteria.

Objective responses were obtained in patients with advanced and/or metastatic cancers refractory to previous treatments.

In particular treatment with this method has shown responses in cancer patients with advanced and/or metastatic disease, which exhibited progressive disease after having been previously treated with established therapies.

More generally, the invention involves the use of a muscle protector in conjunction with aplidine therapy. We have found in particular that carnitine is beneficial in treating myotoxicity that is associated with chemotherapy with the experimental drug aplidine. In Phase I trials when using a 24-hour infusion of aplidine, 4500 mcg/m² every week, then 6000 mcg/m² every second week, some subjects experienced some form of muscular and skeletal toxicity characterized by muscle cramping, pain and myopathic weakness. They also showed measurable increases in serum creatine kinase, an indicator of muscle breakdown and damage. When L-carnitine 4.5 g/day was added to the therapy (at doses of 1.5 g three times daily), or 0.1mg/kg (at doses of 0.033 mg 3 times daily) patients were able to tolerate doses of aplidine up to 6000 mcg/m 2 every second week. The beneficial effect, as seen in normal creatine kinase values, lasted throughout the study period of at least 13 weeks. Therefore, muscle protectors such as L-carnitine is a useful myoprotector for patients being treated with aplidine or an aplidine analogue.

A preferred method of this invention thereto involves identifying cancer patients who have been treated for cancer, particularly patients who have received chemotherapy, and treating them with an aplidine compound, specially aplidine.

The present PCT application claims priority from earlier patent applications. We specifically incorporate them by reference, especialy where there is disclosure not carried forward to the present specification and where that disclosure might be relevant to the present invention.

### DESCRIPTION OF THE DRAWINGS

Figures 1 and 2 show the relationship of muscle enzyme changes for two patients in relationship to administration of aplidine and L-carnitine.
Figure 3 shows the evolution of acylcarnitines for aplidine and aplidine-carnitine.

### EXAMPLES OF THE INVENTION

### Example 1

A phase I and pharmacokinetic study of aplidine, given as a 24h continuous infusion every other week in patients with solid tumours and non-Hodgkin lymphoma

| Patient Characteristics | | | |
|---|---|---|---|
| Number of patients | 43 | Tumour type | |
| Median age, years (ranges) | 52 (18-71) | Lung | 6 |
| ECOG performed status | | Colorectal | 8 |
| 0 | 19 | Kidney | 5 |
| 1 | 21 | Breast | 4 |
| 2 | 2 | Pancreas | 4 |
| Prior radiotherapy | 27 | Lymphoma | 3 |
| Prior chemotherapy (No. regimens) | | Ovary | 2 |
| 1 | 7 | Thyroid | 3 |
| 2 | 5 | Bone | 1 |
| ≥3 | 29 | Melanoma | 1 |
| | | Prostate | 1 |
| | | Uterus | 1 |
| | | Mesothelioma | 1 |
| | | Gastric | 1 |
| | | Other | 2 |

| Patient Accrual and Dose Escalation | | | |
|---|---|---|---|
| Dose level | Dose (mcg/m²/2wks) | No. patients | No. Cycles (range) |
| I | 200 | 3 | 5 (1-3) |
| II | 400 | 3 | 6 (2-2) |
| III | 800 | 3 | 9 (2-4) |
| IV | 1600 | 6 | 11 (1-2) |
| V | 3200 | 3 | 5 (1-2) |
| VI | 4000 | 3 | 8 (2-4) |
| VII | 5000 | 3 | 6 (2-2) |
| VIII | 6000 | 12 | 26 (1-6+) |
| IX | 7000 | 7 | 12 (1-4+) |
| Total | | | |
| *cycle definition: 2bi-weekly infusions | | | |

| Worst Toxicities Per Patient | | | | | | | | | |
|---|---|---|---|---|---|---|---|---|---|
| Dose level | 200 | 400 | 800 | 1600 | 3200 | 4000 | 5000 | 6000 | 7000 |
| No. patients | 3 | 3 | 3 | 6 | 3 | 3 | 3 | 12 | 7 |
| Nausea/ | 2 | 1 | - | 4 | (1) | 2 | 1 | 2 | 1 |
| vomiting G2 (G3) | | | | | | | | | |
| Flushing G1 | - | - | 1 | - | 1 | 1 | - | - | 1 |
| Asthenia G2 (G3) | - | 2 | - | 2(1) | 1 | 1 | (1) | 6 | 2 |
| Muscle cramps G1+G2 | - | - | 2 | 1 | 2 | 2 | 2 | 1 | - |
| Muscle pain G1 (G2) | - | - | - | - | (1) | 2 | 1 | 1(2) | 1 |
| Muscle weakness G1 (G2) | - | - | - | - | - | - | 1 | 1(1) | - |
| CPK elevation | - | - | - | - | - | - | (1) [1] | 1(1) | 1 |
| (G2 (G3) [G4] | | | | | | | | | |
| Transaminitis G2 (G3) | 1 | - | - | (1) | - | - | 1 | - | 1 |
| Hypertension G2 | - | 1 | - | - | - | - | - | - | - |
| Neutrophenia G4 | - | - | - | - | - | - | - | - | 1 |
| Pain central | - | - | - | - | - | 2 | - | - | - |
| catheter G2 | | | | | | | | | |

### Characterisation of Muscular Toxicity (DLT)

Pat #27 - Male patient with medullary thyroid carcinoma treated at 6000 µg/m² weekly had symptomatic G3 CPK with G2 muscular pain. Toxicity recovered within 3 weeks after treatment discontinuation.

3 patients *(5000 and 6000 µg*/*m*^{*2*}*)* experienced a minor elevations of CPKs (≥G2), consisting of CPK MM (muscle) increase with no significant elevation of SPK MB (heart). A parallel elevation of the aldolase level was observed. Signs of improvement by using Carnitine supplements as skeletal muscle protectors are being reported. Muscle biopsies were performed in 2 patients; E/M: partial disappearance of thick filaments of myosin.

### Pharmacokinetic Data

Aplidine appears to have a dose-linear PK profile (within the constraints imposed by the low sample size)
Relatively high plasma CL: median (quartiles) value 252 (192-415 mL/min/m²)
High interpatient CL variability (coefficient of variation of CL 62%) Intermediate to long t ½ with a median (quartiles) value of 23.8 (15.7-35.0 h)
Wide distribution, median (quartiles) Vss of 413 (274-638 L/m²) Preliminary compartmental analysis: plasma profiles are best fit by a first-order 2-compartment model with a rapid initial (median half-life 0.64 h) and a longer terminal phase (median half-life 25.8 h)

### Aplidine Mytotoxicity Relationship with Pharmacokinetics

Muscular toxicity has appeared only at high doses and exposures after 24 h infusion
Cmax values after 1 h infusion are already higher than those after 24 h infusion. Hence, a Cmax relationship may be ruled out
The AUC values in the patients with myotoxicity are high but not the maximum
It affected patients with high, sustained plasma concentrations of aplidine. The 3 patients with clear muscular toxicity had t ½ in excess of 44 h as compared to a median t ½ of 25.8 h after 24 h infusion

### Conclusions

Drug induced muscular changes (expected to be the dose limiting toxicity), reported from dose level number III onwards (1800 mcg/m² to 5000 mcg/m²) is dose limiting toxicity at 6000 mcg/m² (1/9 pts) Antitumour activity has been also noted in patients with NHL and renal carcinoma

The study is now investigating the feasibility of 6000-7000 mcg/m² every other week by using carnitine supplements as skeletal muscle protectors.

### Example 2

Phase I studies of aplidine revealed aplidine myotoxicity.

### Clinical features

The presentation is variable among patients. Mild cases have muscle cramps (at doses from 3200 mcg/m2 every 2 weeks), while in more severe cases the symptoms are associated to reversible increases in creatin-kinase (CK) reaching up to grade 3. In the dose-limiting cases there is weakness of proximal distribution. The effect has a delayed onset, appearing after 3 to 8 (median 4) infusions of the drug.

### Pathological features

Light microscopy: just minimal necrosis or no changes at all (in most biopsied patients) or type II fiber atrophy (in a patient with gastric adenocarcinoma and concomitant long term 10 mg/d prednisone). Electron microscopy: a specific accumulation of glycogen and autophagocytic vacuoles, being the most important change the disappearance of thick filaments.

### Relationship to exposure

The maximum concentrations (Cmax) observed after 1 h infusion, before even hints of myotoxicity were found, were higher than those after doses related to myotoxicity 24 h infusion ruling out a Cmax relationship.

The area under the curve (AUC) values in patients with myotoxicity tend to be high but not uniformly and not the maximum. Patients with dose-limiting myotoxicity had the longest terminal half-lives with the exception of a patient who received just 2 aplidine infusions, i.e. a treatment too short to be evaluable for myotoxicity. Another patient with myotoxicity had a short half life. Probably the relatively high AUC in some patients with muscular toxicity reflected the long half life. Hence, aplidine myotoxicity appears related to prolonged exposure rather than high exposure or concentrations.

### Muscle enzymes changes

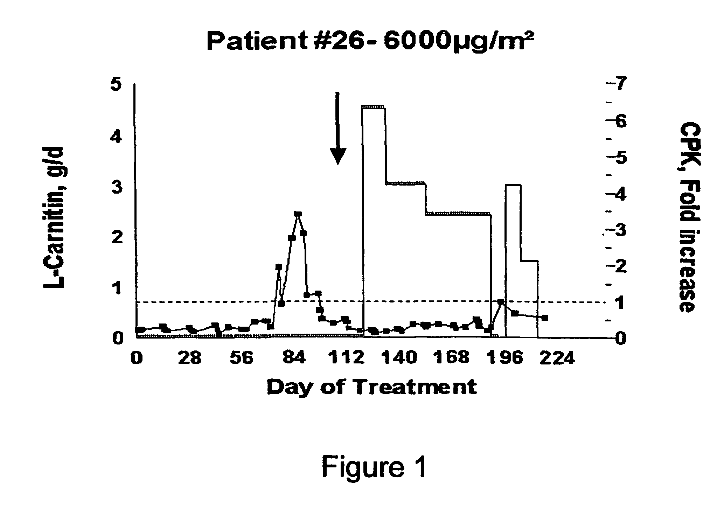

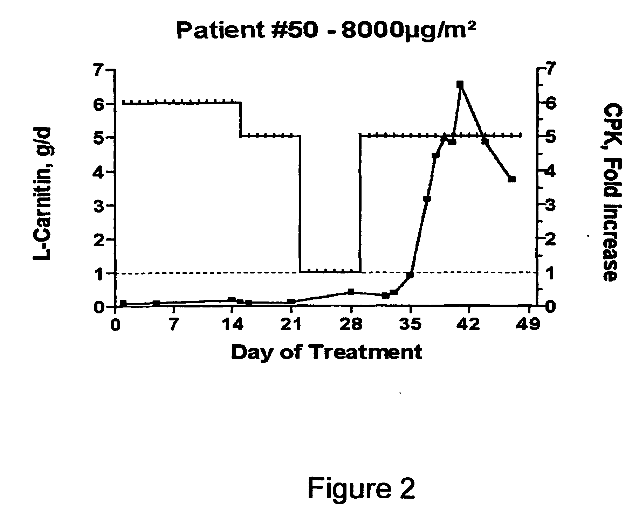

Examples of the relationship to aplidine and L-carnitine administration are shown in Figures 1 and 2. For both figures, aplidine was given every 14 days starting from Day 1. For Figure 1, the arrow indicates omitted aplidine dose.

### Corrective measures

L-carnitine at a dose of 1.5 grams 3 times per day or 0.033 g/kg 3 times per day was started in all patients of study APL-A-003-98. In the study, 4 patients with prior skeletal muscle toxicity were able to continue treatment with no skeletal muscle symptoms and just transient grade 1 CK increases. Among the 11 patients receiving L-carnitine prophylaxis from the beginning in the above study there was just an asymptomatic G1 increase in CK. Two patients had symptomatic increases in CK (grade 3 associated to weakness & grade 1 associated to asthenia, respectively) after treatment at reduced doses (1 g for a week due to an administrative problem and irregular bad compliance respectively).

L-carnitine at the doses used in this study is well tolerated. The only toxicity reported to date is the presence of grade 1 abdominal discornfort and diarrhea. The dose may be decreased to manage the above effects. Initial graphical analysis of patients with increases in CK after decreased L-carnitine dose suggest that the dose should not be decreased below 3.5 grams per day.

In all ongoing studies a maximum tolerated dose (MTD) and a recommended dose (RD) without prophylactic L-carnitine (only allowed on a therapeutic basis) would be defined. Later in the study, systematic L-carnitine prophylaxis will be started and a new MTD and RD will be defined.

The possibility of tumor protection by L-carnitine was evaluated using a panel of 25 human tumor cell lines. Initial results are compatible with a lack of effect of L-carnitine on the antitumor activity of aplidine.

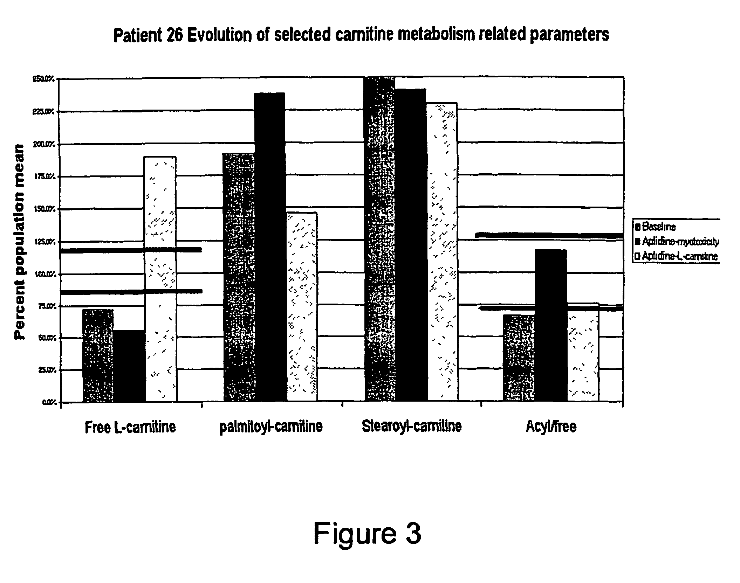

Figure 3 shows the evolution of acylcarnitines, where the thick lines represent 95% CI for the normal population for the respective parameter.

### Acylcarnitine profile

It was measured by tandem mass spectrometry in plasma from a patient with clear toxicity (cramps + increased CK +weakness) at baseline, during toxicity and after aplidine treatment continued under L-carnitine protection. At baseline, there was decreased free L-carnitine, increased palmitoyl and steraroylcarnitine. During myotoxicity while on aplidine alone, free L-carnitine decreased while palmitoylcarnitine increased and stearoylcarnitine was stable L-Carnitine increased serum free carnitine up to supranormal values, while decreasing palmitoylcarnitine. Stearoylcarnitine was stable. Values for long chain acylcarnitines were less than half the diagnostic cutoff for CPT-II deficiency. Hence, this was not the underlying molecular defect (or at least not the only defect) present in this patient.

### Conclusions

The dose-limiting factor for aplidine in 4 Phase I trials has been skeletal muscle toxicity. L-carnitine was assessed as a myoprotector for use in patients. The available clinical data demonstrates that L-carnitine prophylaxis enabled to increase aplidine MTD-RD by 33% and 40% respectively in the 24 h every other week infusion study, being the new dose-limiting factor non-muscular.

Phase II studies will be initiated using a RD of aplidine of 7 mg/m² and L-carnitine at an initial dose of 0.1 g/kg /day divided in 3 portions.

## Claims

1. The use of an aplidine compound in the preparation of a medicament for the treatment of a cancer by administering aplidine or an aplidine analog and a skeletal muscle protector, selected from L-carnitine, racemic carnitine, precursors and derivatives of L-carnitine, and acetylcarnitine.

2. The use of a skeletal muscle protector, selected from L-carnitine, racemic carnitine, precursors and derivatives of L-carnitine, and acetylcarnitine in the preparation of a medicament for the treatment of a cancer by administering aplidine or an aplidine analogue and said skeletal muscle protector.

3. The use of an aplidine compound and a skeletal muscle protector, selected from L-carnitine, racemic carnitine, precursors and derivatives of L-carnitine, and acetylcarnitine in the preparation of medicaments for the treatment of a cancer by administering aplidine or an aplidine analogue and said skeletal muscle protector.

4. A use according to claim 1, 2 or 3, where the aplidine compound and muscle protector are administered as separate compositions with different dosing regimes.

5. A use according to any preceding claim, where the aplidine compound is aplidine.

6. A use according to claim 5, wherein the dosing of aplidine is in accordance with one of the following protocols;
24 hour infusion weekly for three weeks, followed by one week rest;
biweekly 24 hour infusion;
1 hour infusion weekly for three weeks every 4 weeks;
daily 1 hour IV infusion x 5 days q 3 weeks; and
3 hour infusion every other week.

7. A use according to any preceding claim, wherein the muscle protector is L-carnitine.

8. A use according to claim 7, wherein the L-carnitine is administered daily as divided doses.

9. The use according to any preceding claim, where the patient has already received treatment for cancer disease and the tumour is refractory.

## Patentansprüche

1. Verwendung einer Aplidin-Verbindung bei der Herstellung eines Arzneimittels zur Behandlung eines Krebses durch Verabreichung von Aplidin oder einem Aplidin-Analogon und einem Skelettmuskulatur-Schutzstoff, ausgewählt aus L-Carnitin, racemischem Carnitin, Präkursoren und Derivaten von L-Carnitin und Acetylcarnitin.

2. Verwendung eines Skelettmuskulatur-Schutzstoffs, ausgewählt aus L-Carnitin, racemischem Carnitin, Präkursoren und Derivaten von L-Carnitin und Acetylcarnitin bei der Herstellung eines Arzneimittels zur Behandlung eines Krebses durch Verabreichung von Aplidin oder einem Aplidin-Analogon und einem Skelettmuskulatur-Schutzstoff

3. Verwendung einer Aplidin-Verbindung und eines Skelettmuskulatur-Schutzstoffs, ausgewählt aus L-Carnitin, racemischem Carnitin, Präkursoren und Derivaten von L-Carnitin und Acetylcarnitin bei der Herstellung von Arzneimitteln zur Behandlung eines Krebses durch Verabreichung von Aplidin oder einem Aplidin-Analogon und einem Skelettmuskulatur-Schutzstoff.

4. Verwendung nach Anspruch 1, 2 oder 3, worin die Aplidin-Verbindung und der Muskulatur-Schutzstoff als getrennte Zusammensetzungen mit unterschiedlichen Dosierungsregimen verabreicht werden.

5. Verwendung nach einem der vorangehenden Ansprüche, worin die Aplidin-Verbindung Aplidin darstellt.

6. Verwendung nach Anspruch 5, worin die Dosierung von Aplidin gemäß einem der folgenden Protokolle erfolgt:
Wöchentliche 24-Stunden-Infusion für drei Wochen, gefolgt von einer Woche Pause;
zweiwöchentliche 24-Stunden-Infusion;
wöchentliche 1-Stunden-Infusion für drei Wochen alle 4 Wochen;
tägliche 1-Stunden-iv-Infusion x 5 Tage alle 3 Wochen; und
3-Stunden-Infusion jede andere Woche.

7. Verwendung nach einem der vorangehenden Ansprüche, worin der Muskulatur-Schutzstoff L-Carnitin darstellt.

8. Verwendung nach Anspruch 7, worin das L-Carnitin täglich in aufgeteilten Dosen verabreicht wird.

9. Verwendung nach einem der vorangehenden Ansprüche, worin der Patient bereits wegen einer Krebserkrankung behandelt wurde und der Tumor refraktär ist.

## Revendications

1. Utilisation d'un composé d'aplidine dans la préparation d'un médicament pour le traitement d'un cancer en administrant de l'aplidine ou un analogue d'aplidine et un protecteur de muscles squelettiques, sélectionné parmi la L-carnitine, la carnitine racémique, des précurseurs et des dérivés de L-carnitine et l'acétyl-carnitine.

2. Utilisation d'un protecteur de muscles squelettiques, sélectionné parmi la L-carnitine, la carnitine racémique, des précurseurs et des dérivés de L-carnitine et l'acétyl-carnitine dans la préparation d'un médicament pour le traitement d'un cancer en administrant de l'aplidine ou un analogue d'aplidine et ledit protecteur de muscles squelettiques.

3. Utilisation d'un composé d'aplidine et d'un protecteur de muscles squelettiques, sélectionné parmi la L-carnitine, la carnitine racémique, des précurseurs et des dérivés de L-carnitine et l'acétyl-carnitine dans la préparation de médicaments pour le traitement d'un cancer en administrant de l'aplidine ou un analogue d'aplidine et ledit protecteur de muscles squelettiques.

4. Utilisation selon la revendication 1, 2 ou 3, où le composé d'aplidine et le protecteur musculaire sont administrés en tant que compositions séparées avec des schémas de dosage différents.

5. Utilisation selon l'une quelconque des revendications précédentes, où le composé d'aplidine est de l'aplidine.

6. Utilisation selon la revendication 5, où le dosage de l'aplidine est conforme à l'un des protocoles suivants;
perfusion de 24 heures toutes les semaines pendant trois semaines, suivie par une semaine de repos;
perfusion bihebdomadaire de 24 heures;
perfusion d'une heure toutes les semaines pendant trois semaines toutes les quatre semaines;
perfusion IV quotidienne pendant une heure x 5 jours toutes les 3 semaines; et
perfusion de 3 heures toutes les deux semaines.

7. Utilisation selon l'une quelconque des revendications précédentes, où le protecteur musculaire est de la L-camitine.

8. Utilisation selon la revendication 7, où la L-carnitine est administrée tous les jours en doses divisées.

9. L'utilisation selon l'une quelconque des revendications précédentes, où le patient a déjà reçu un traitement pour une maladie cancéreuse et où la tumeur est réfractaire.
